# EUROPEAN PATENT APPLICATION

(11) **EP 4 723 124 A1**
(43) Date of publication of application: **08.04.2026**
(21) Application number: 25206645.1
(22) Date of filing: 03.10.2025
(51) Int. Cl.: G16H 20/40, G16H 50/20, G16H 50/70, G16H 70/20

(54) **USING MACHINE-LEARNING LANGUAGE PROCESSING MODEL FOR RADIOTHERAPY TREATMENT PLANNING**

(30) Priority: 04.10.2024 US 202418907368
(71) Applicant: Siemens Healthineers International AG, 6312 Steinhausen (CH)
(72) Inventor: PELTOLA, Jarkko, 04300 Tuusula (FI); SABEL, Martin, 6332 Hagendorn (CH); KUUSELA, Esa, 02320 Espoo (FI); CZEIZLER, Elena, 00390 Helsinki (FI); HAUTALA, Ismo, 02600 Espoo (FI); DONNELLY, Kellee, 00550 Helsinki (FI)
(74) Representative: Mathisen & Macara LLP

(57) **Abstract**

Disclosed herein are methods and systems for using machine-learning language processing models to provide contextual data while a medical professional is using a radiotherapy treatment planning user interface to generate a treatment plan for a patient. One method comprises monitoring 202, by at least one processor, a radiotherapy treatment planning user interface comprising an interaction interface to identify one or more radiotherapy planning attributes associated with a radiotherapy treatment of a patient via a radiotherapy treatment planning computer model; receiving 204, by at least one processor from the radiotherapy treatment planning computer model, an indication associated with the radiotherapy planning attribute; responsive 206 to the indication satisfying a threshold: executing 210, by at least one processor, a machine-learning language processing model to generate contextual data associated with the one or more radiotherapy planning attributes; and presenting 212, by at least one processor, the contextual data within the radiotherapy treatment planning user interface.

## Description

### TECHNICAL FIELD

This application relates generally to generating a radiotherapy treatment plan contextual data using a machine learning language processing model.

### BACKGROUND

Radiotherapy or radiation therapy (RT) is one of the main modalities used in cancer treatment, and RT treatment planning (RTTP) is a complex process that contains specific guidelines, protocols, and instructions adopted by different medical professionals, such as clinicians, medical device manufacturers, and the like. Medical professionals strive to deliver the safest and most effective treatment to patients.

The RTTP creation typically involves a collaboration of multiple professionals and an automated computer model (e.g., treatment planner or plan optimizer). The initial treatment plan is usually created based on the best available information at the time, tailored from standard treatment protocols to suit the individual patient. Specifically, various attributes are inputted by a medical professional into an interface of the treatment planner, where the computer model optimizes the RTTP using those attributes. Then, the medical professionals review various attributes of a treatment plan and may perform multiple rounds of additional interaction with the computer model before approving the plan. Therefore, the medical planner's experience and subjective knowledge are crucial in the creation of RTTP.

RTTP generally requires specialized knowledge, necessitating thorough training for new clinic personnel before they can effectively use the planning software. This training is essential when staff start using a new planning system or when the software is updated to support new optimization or treatment techniques, ensuring alignment with current clinical practices. Achieving full expertise takes even longer, and the rapidly evolving RT domain requires continuous learning of new aspects of treatment planning. Educating new or junior staff members adds to the workload of senior personnel, which is highly undesirable.

### SUMMARY

There are no effective products aimed at solving the above-identified problems. While treatment planning systems may include help files, these are limited to purely technical instructions provided by the software manufacturer. For example, some RTTP computer models allow adding a document with application instructions and/or allow providing access to this document with a single mouse click. However, these solutions are static and do not substitute for real training. Moreover, treatment planners are required to identify the right information in the right section of the electronic document, which is a burdensome and tedious task. Moreover, this conventional method heavily relies on each treatment planner's subjective understanding of the knowledge included within static documents, which is highly undesirable and inconsistent throughout different clinics.

Embodiments disclosed herein include computing systems that execute software components for machine-learning architecture functions, including an artificial intelligent assistant ("AI assistant" or "AI agent"), to improve treatment planning process and to improve the efficiency and efficacy of treatment planning. Using the methods and systems discussed herein, a server may employ various AI tools and techniques to generate real-time or near-real-time data that can help with the treatment planning process. The data provided by the AI agent may be tailored to the treatment planner, patient, and/or the clinic so that the needs of the treatment planner are satisfied at any stage of the treatment planning process.

The methods and systems discussed herein provide a sophisticated system leveraging machine learning language processing models (e.g., large language models (LLM)) to enhance radiotherapy planning by providing tailored and context-specific guidance to treatment planners. Unlike current chatbots trained on generic data, the machine learning language processing models discussed herein may be fine-tuned with specific product information, physician or patient-specific data, and/or clinic-specific protocols, allowing it to offer precise advice and support, and not generic responses as provided by conventional/current systems. The AI agent discussed herein may operate by assisting treatment planners with checking for missing information, ensuring all necessary steps are followed, and providing specific recommendations based on patient data and clinical practices. By integrating multiple machine learning language processing models, the AI agent may retrieve and process relevant information, enhancing the accuracy and relevance of the guidance provided and ultimately improving the quality and efficiency of radiotherapy planning while fostering planner expertise over time.

The technical solution provided via the methods and systems discussed herein utilizes a machine learning language processing model-powered AI agent that acts as an "education agent" to minimize the need for human instructors to train RT personnel on treatment planning systems. Therefore, the AI agent may offer a conversational interface for the treatment planning systems, enabling planners to ask questions and engage in discussions. The training agent would provide instructions, explanations, and recommendations, enhancing the learning experience and support for the treatment planners. The machine learning language processing model may be trained to, for example, provide follow-up questions regarding any input provided by the medical professional. That way, the machine learning language processing model may have a pseudo conversation with the medical professional until the machine learning language processing model identifies the attribute and enough information to generate (or instruct others to generate) the patient's treatment plan.

In one embodiment, a method comprises monitoring, by at least one processor, a radiotherapy treatment planning user interface comprising an interaction interface to identify one or more radiotherapy planning attributes associated with a radiotherapy treatment of a patient via a radiotherapy treatment planning computer model; receiving, by at least one processor from the c, an indication associated with the radiotherapy planning attribute; responsive to the indication satisfying a threshold: executing, by at least one processor, a machine-learning language processing model to generate contextual data associated with the one or more radiotherapy planning attributes; and presenting, by at least one processor, the contextual data within the radiotherapy treatment planning user interface - for example by an electronic or computer display, or by an audio output device.

The indication may correspond to missing data.

The indication may correspond to the one or more radiotherapy planning attributes causing at least one attribute of the radiotherapy treatment violate a plan objective.

The presenting the contextual data may correspond to outputting an electronic document corresponding to at least one of an instruction manual, a setting or guideline specific to the radiotherapy treatment planning computer model, or a treatment planning guideline.

The contextual data may be specific to a clinic implementing the radiotherapy treatment of the patient.

The contextual data may comprise a visualization of the radiotherapy treatment of the patient.

The method may further comprise recalibrating, by the processor, the machine-learning language processing model using an input corresponding to a quality of the contextual data.

In another embodiment, a system comprises a non-transitory computer-readable medium having non-transitory instructions that when executed, cause a processor to: monitor a radiotherapy treatment planning user interface comprising an interaction interface to identify one or more radiotherapy planning attributes associated with a radiotherapy treatment of a patient via a radiotherapy treatment planning computer model; receiving, by at least one processor from the radiotherapy treatment planning computer model, an indication associated with the radiotherapy planning attribute; responsive to the indication satisfying a threshold: execute a machine-learning language processing model to generate contextual data associated with the one or more radiotherapy planning attributes; and present the contextual data within the radiotherapy treatment planning user interface - for example by an electronic or computer display, or by an audio output device.

The indication may correspond to missing data.

The indication may correspond to the one or more radiotherapy planning attributes causing at least one attribute of the radiotherapy treatment violate a plan objective.

The presenting the contextual data may correspond to outputting an electronic document corresponding to at least one of an instruction manual, a setting or guideline specific to the radiotherapy treatment planning computer model, or a treatment planning guideline.

The contextual data may be specific to a clinic implementing the radiotherapy treatment of the patient.

The contextual data may comprise a visualization of the radiotherapy treatment of the patient.

The instructions may further cause the processor to recalibrate the machine-learning language processing model using an input corresponding to a quality of the contextual data.

In another embodiment, a system comprises a machine-learning language processing model; and a processor in communication with the machine-learning language processing model, the processor configured to monitor a radiotherapy treatment planning user interface comprising an interaction interface to identify one or more radiotherapy planning attributes associated with a radiotherapy treatment of a patient via a radiotherapy treatment planning computer model; receiving, by at least one processor from the radiotherapy treatment planning computer model, an indication associated with the radiotherapy planning attribute; responsive to the indication satisfying a threshold: execute a machine-learning language processing model to generate contextual data associated with the one or more radiotherapy planning attributes; and present the contextual data within the radiotherapy treatment planning user interface - for example by an electronic or computer display, or by an audio output device.

The indication may correspond to missing data.

The indication may correspond to the one or more radiotherapy planning attributes causing at least one attribute of the radiotherapy treatment violate a plan objective.

The presenting the contextual data may correspond to outputting an electronic document corresponding to at least one of an instruction manual, a setting or guideline specific to the radiotherapy treatment planning computer model, or a treatment planning guideline.

The contextual data may be specific to a clinic implementing the radiotherapy treatment of the patient.

The contextual data may comprise a visualization of the radiotherapy treatment of the patient.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates components of a system implementing an AI-enabled treatment planning system in accordance with an embodiment.
**FIG. 2** shows an operational workflow of a method performed in hardware and software computing components that host and execute an AI-enabled treatment planning system in accordance with an embodiment.

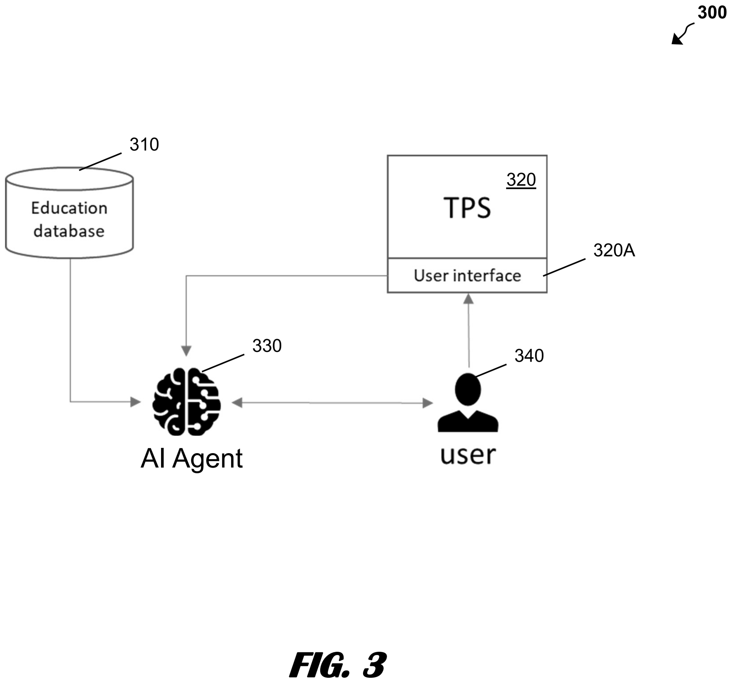

**FIG. 3** illustrates a non-limiting example of a data flow within an AI-enabled treatment planning system in accordance with an embodiment.

### DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are to be considered within the scope of the subject matter disclosed herein. Other embodiments may be used, and/or other changes may be made without departing from the spirit or scope of the present disclosure. The illustrative embodiments described in the detailed description are not meant to limit the subject matter presented.

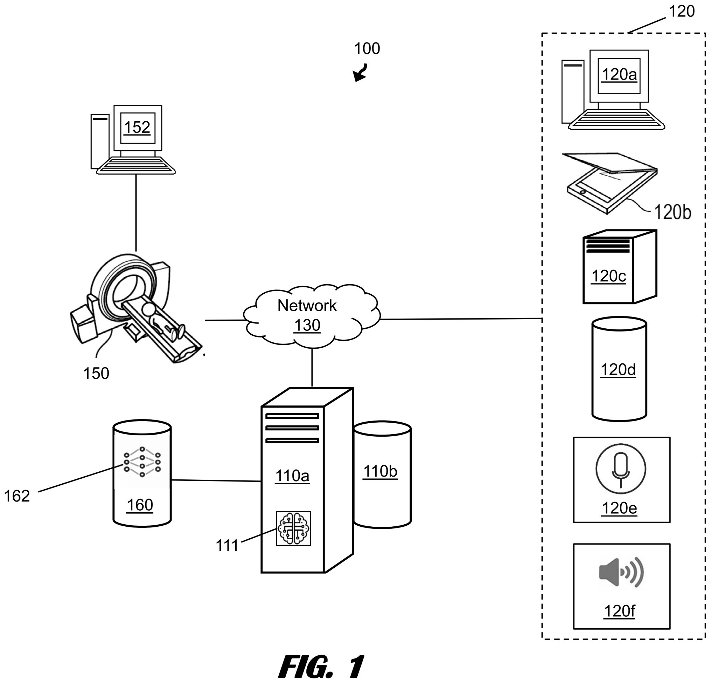

**FIG. 1** illustrates components of a system **100** for an AI-enabled treatment planning system, according to an embodiment. The system **100** may include an analytics server **110a,** a system database **110b,** a machine learning language processing model **111,** end-user devices **120a-120f** (collectively end-user devices **120),** a medical device **150,** a medical device computer **152,** a database **160,** and a radiotherapy plan optimizer **162.** Various components depicted in **FIG. 1** may belong to a radiation therapy treatment clinic at which patients may receive radiation therapy treatment, in some cases via one or more radiation therapy machines (e.g., the medical device **150).**

The system **100** is not confined to the components described herein and may include additional or other components, not shown for brevity, which are to be considered within the scope of the embodiments described herein.

The above-mentioned components may be connected to each other through one or more networks **130.** Examples of the network **130** may include, but are not limited to, private or public local-area networks (LAN), wireless local-area networks (WLAN), metropolitan-area networks (MAN), wide-area networks (WAN), and the Internet. The network **130** may include wired and/or wireless communications according to one or more standards and/or via one or more transport mediums. The communication over the network **130** may be performed in accordance with various communication protocols such as Transmission Control Protocol and Internet Protocol (TCP/IP), User Datagram Protocol (UDP), and IEEE communication protocols. In one example, the network **130** may include wireless communications according to Bluetooth specification sets or another standard or proprietary wireless communication protocol. In another example, the network **130** may also include communications over a cellular network, including, e.g., a GSM (Global System for Mobile Communications), CDMA (Code Division Multiple Access), or EDGE (Enhanced Data for Global Evolution) network.

The analytics server **110a** may generate and display an electronic platform configured to interface a user with the radiotherapy plan optimizer **162** and/or (indirectly with) the machine learning language processing model **111** and for receiving patient information (via various sources) and visualization preferences/instructions. The analytics server **110a** may then output the results of the execution of the machine learning language processing model **111** and the radiotherapy plan optimizer **162.** In some embodiments, the platform and/or the interaction interface of the platform may be populated via the radiotherapy plan optimizer **162** itself.

The electronic platform may include graphical user interfaces (GUI) displayed on each of the end-user devices **120,** the medical device **150,** and/or the medical device computer **152.** An example of the electronic platform generated and hosted by the analytics server **110a** may be a web-based application or a website configured to be displayed on different electronic devices, such as mobile devices, tablets, personal computers, and the like.

The platform hosted on the analytics server **110a** or another device of the system **100** includes collaboration software accessible to the user devices **120** of the participating members, such that multiple medical professionals can collaborate and view the visualizations provided by the analytics server **110a.** The collaboration software may include any type of software facilitating user-group collaborations, which may include live interaction software (e.g., teleconferencing software) or asynchronous collaborations (e.g., online postings). Non-limiting examples of collaboration software include MS Teams^{®}, Skype^{®}, WebEx^{®}, Slack^{®}, and Twilio^{®}, among others. The collaboration software may also facilitate communication between one or more medical professionals and the analytics server **110a** and/or the radiotherapy plan optimizer **162.** For instance, the platform provided or hosted by the analytics server **110a** may include an input element (e.g., visual, textual, or auditory), allowing users (e.g., one or more medical professionals) to input their desired treatment plan attributes. The platform may also display predicted outputs by the radiotherapy plan optimizer **162.** As described herein, the analytics server **110a** may also display outputs predicted by the machine learning language processing model **111.**

The information displayed by the analytics server **110a** of the electronic platform can include any contextual data associated with a user's input while generating a treatment plan. For example, the analytics server may display any data needed to generate a treatment plan for one or more patients. Non-limiting examples may include data associated with a patient to be treated (e.g., plan objectives) or visualization attributes (e.g., what the medical professional desires to view), missing data, incorrect data, contextual data associated with how to operate the radiotherapy plan optimizer 162, and the like.

The analytics server **110a** may be any computing device comprising a processor and non-transitory machine-readable storage capable of executing the various tasks and processes described herein. The analytics server **110a** may employ various processors, such as central processing units (CPU) and graphics processing units (GPU), among others. Non-limiting examples of such computing devices may include workstation computers, laptop computers, server computers, and the like. While the system **100** includes a single analytics server **110a,** the analytics server **110a** may include any number of computing devices operating in a distributed computing environment, such as a cloud environment.

End-user devices **120** may be any computing device comprising a processor and a non-transitory machine-readable storage medium capable of performing the various tasks and processes described herein. Non-limiting examples of an end-user device **120** may be a workstation computer, laptop computer, tablet computer, or server computer. In operation, various users may use end-user devices **120** to access the GUI operationally managed by the analytics server **110a.** Specifically, the end-user devices **120** may include a clinic computer **120a,** a clinic server **120b,** and medical professional devices **120c,** which may include any electronic devices operated by members of the Tumor Board, medical professionals, and scientists that access and review various types of patient-related treatment data and RTTPs for the patient, among other types of data and information exchanges.

In a non-limiting example, multiple medical professionals may operate the medical professional devices **120c** to review patient-related treatment data to develop a consensus on a treatment for the patient. Even though referred to herein as "end-user" devices, these devices may not always be operated by end-users. For instance, the clinic server **120b** may not be directly used by an end-user. However, the results stored on the clinic server **120b** may be used to populate various GUIs accessed by an end-user via the medical professional device **120c.** Patient-related information generated by the various types of devices in system 100, outside the context of the AI treatment planning agent, may be stored within database **100b.** The stored patient data may be referenced by the analytics server **110a** for training the machine learning language processing model **111.**

The medical device **150** may be a radiation therapy machine configured to implement a patient's radiotherapy treatment. The medical device **150** may also be in communication with a medical device computer **152** that is configured to display various GUIs discussed herein. For instance, the analytics server **110a** may display the results predicted by the radiotherapy plan optimizer **162** onto the computing devices described herein.

The machine learning language processing model **111** may be stored in the system database **110b.** The machine learning language processing model **111** may be configured or trained to automatically generate text, image, or video responses based on inputs received at a user interface or other types of inputs (e.g., speech captured at a conference room microphone or microphone of an end-user device **120).**

The user-provided inputs include, for example, text inputs entered by one or more medical professionals or audio signals containing speech audio of the medical professional captured by microphones of the user devices **120** or conference room. The analytics server **110a** may include or invoke Automated Speech Recognition (ASR) software that converts speech audio to written text. The ASR software comprises a machine-learning architecture trained to detect portions or frames of the audio signal containing the speech audio of a member speaker. The ASR also comprises and applies Natural Language Processing (NLP) layers of the machine-learning architecture that generates text-based output from the portions of the audio signal containing the detected speech audio. The text generated by the ASR may be fed as an input to the machine learning language processing model **111.** In some embodiments, the ASR may be incorporated within (e.g., be a feature of) the machine learning language processing model **111.**

In some embodiments, the analytics server **110a** may execute the radiotherapy plan optimizer **162** to generate one or more treatment attributes for an RTTP complying with any radiation therapy plan objectives based on patient attributes of a patient for which the radiotherapy treatment plan is being generated. The radiotherapy plan optimizer **162** can be stored in the database **160.** The radiotherapy plan optimizer **162** can generate the one or more treatment attributes, for example, by iteratively calculating the one or more treatment attributes where, with each iteration, the radiotherapy plan optimizer **162** can revise the one or more treatment attributes of the RTTP in accordance with a cost value.

The analytics server **110a** may deploy the radiotherapy plan optimizer **162** to generate an RTTP for a patient based on patient attributes and one or more treatment attributes received from one or more end-user devices **120.** The radiotherapy plan optimizer **162** may iteratively calculate one or more treatment attributes of the RTTP. For instance, with each iteration, the radiotherapy plan optimizer **162** may generate a candidate RTTP having various attributes. The plan optimizer **162** may then use one or more loss functions to calculate a cost value for the generated candidate RTTP. The cost value may indicate a likelihood of the candidate RTTP violating a set of rules, whether internal and/or external rules. For instance, the cost value may indicate whether the candidate RTTP violates any of the plan objectives. The radiotherapy plan optimizer **162** may analyze the cost value. If needed (e.g., when the cost value satisfies a threshold), the radiotherapy plan optimizer **162** may revise the candidate RTTP and re-execute its loss function to generate a new cost value.

Depending on whether the new cost function is increasing or decreasing, the plan optimizer computer model may revise the candidate RTTP again and recalculate the cost value. The radiotherapy plan optimizer **162** may continue this iterative approach until converging upon an RTTP (or the final RTTP) that has a cost value that satisfies a threshold. In some implementations, the treatment attribute for the patient may also indicate how the radiotherapy treatment may be combined or sequentially implemented with other types of treatment modalities (e.g., surgery, chemotherapy).

The analytics server **110a** can identify the treatment attributes that the radiotherapy plan optimizer **162** determined have a cost value that satisfies the threshold. The analytics server **110a** can present the treatment attributes as an RTTP at the end-user device **120** being accessed by the user, generating the radiotherapy treatment plan. Specifically, the analytics server **110a** may utilize the visualization engine **112** to visualize various attributes of the treatment plan.

In some embodiments, the analytics server **110a** or the end-user device **120** can use the RTTP to automatically control the medical device **150** based on attributes of the RTTP to treat the patient.

The system database **110b** may contain data used to train the machine learning language processing model **111.** For instance, the system database **110b** may include data associated with previously treated patients, such as patient diagnosis data (e.g., tumor data or tumor location), biometric data (e.g., BMI, body weight, height, or various other bodily measurements), and the like. Additionally, the system database **110b** may include visualizations corresponding to the previously treated patients as well. Therefore, the system database **110b** may include all data associated with how the previously treated patients were diagnosed and treated and how the treatment plan was visualized. As described herein, the analytics server **110a** may use the data stored within the system database **110b** to train the machine learning language processing model **111.** The analytics server **110a** may also use the data stored within the database **110b** to visualize a particular patient's visualization itself.

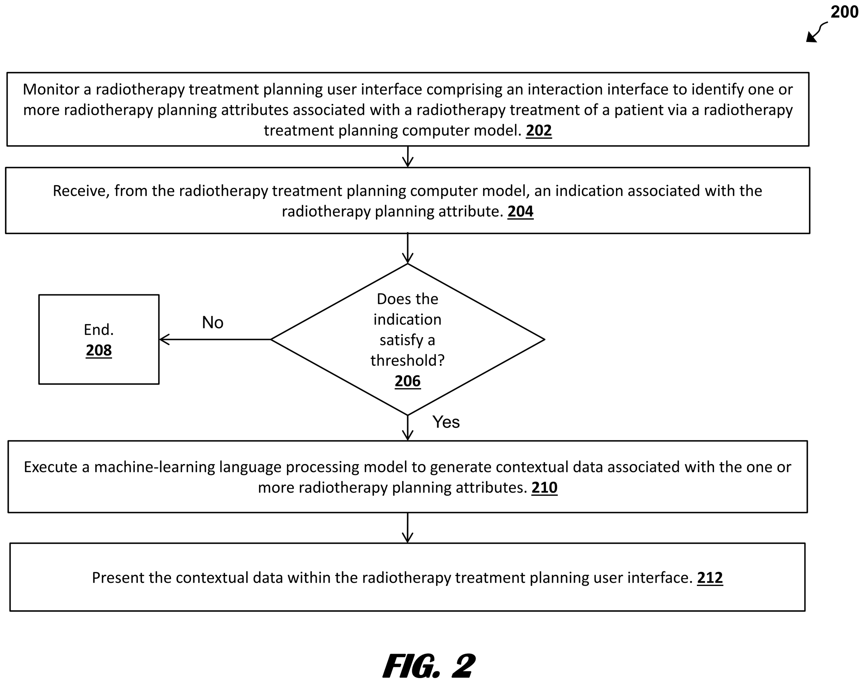

**FIG. 2** shows an operational workflow of method **200** performed in hardware and software computing components that host an AI-enabled treatment planning system in accordance with an embodiment. The method **200** may include steps **202-212.** However, other embodiments may include additional or alternative steps or may omit one or more steps altogether. The method **200** is described as being executed by a server, such as the analytics server described in **FIG. 1****.** However, one or more steps of the method **200** may be executed by any number of computing devices operating in the distributed computing system described in **FIG. 1****.** For instance, one or more computing devices may locally perform some or all of the steps described in **FIG. 2****.**

In the method **200,** the analytics server may use machine learning language processing models in order to provide additional information needed to achieve better results. Specifically, the analytics server may provide contextual data and suggestions regarding the treatment attributes being used by the user, such that the treatment attributes can be revised (by the user) in order to achieve a better treatment plan. The machine learning language processing model's predictions may be outputted using a widget referred to herein as the AI agent or an education agent. The machine learning language processing model may use generative artificial intelligence paradigm that processes a sequence of tokens (e.g., words) to predict the most probable continuation of that sequence to generate the output presented by the AI agent. Typically, the machine learning language processing model discussed herein may be trained on an extensive and diverse set of texts and specific applications and uses a pre-trained foundational model that is fine-tuned for the specific problem at hand.

The machine learning language processing model may not be limited to large language models (LLMs). For instance, in some embodiment, the analytics server may use a multimodal LLM (MM-LLMs) that can integrate various types of information beyond written language, such as medical images and spatially structured data, including organ and target delineations, dose distributions, and field geometry specifications.

For instance, the MM-LLM may convert images into text or process pairs of images and text to generate textual information. For instance, an image-to-text model may convert an image into textual information that can be embedded into the LLM's input token sequence. A text-and-image-to-text model can operate in a more complex setup, where an initial LLM call generates specific questions related to the image (e.g., "have you considered these hot spots in the planning target volume?").

In some embodiments, the MM-LLM may be trained to provide clinically relevant contextual data (e.g., suggestions, guidance, and/or answers) based on the image, to integrate this information with other data. Therefore, the MM-LLM may enable the AI agent to answer detailed clinical questions (e.g., "Does the overlap with the target and the Left Parotid Gland contribute to failing the clinical goal of the mean dose in the Parotid gland?").

At step **202,** the analytics server may monitor a radiotherapy treatment planning user interface comprising an interaction interface to identify one or more radiotherapy planning attributes associated with a radiotherapy treatment of a patient via a radiotherapy treatment planning computer model.

The analytics server may monitor communication between the medical professional (also referred to herein at the user) and the radiotherapy treatment planning computer model. In some embodiment, the radiotherapy treatment planning computer model may have its own user interface (referred to sometimes as the radiotherapy planning user interface) where the user can input various radiotherapy planning attribute using an interaction interface (e.g., input elements, such as upload input elements, text input elements, radio buttons, and the like).

In some embodiments, the analytics server may present a user interface on a computing device operated by a medical professional (e.g., end-user device **120** discussed in **FIG. 1****).** The analytics server may host a platform having various user interfaces that include collaboration software. The analytics server may then transmit the user interface to the computing device over a network. The platform may be used by two or more medical professionals to facilitate discussions regarding treatment considerations and planning (e.g., RTTP). Therefore, the interaction interface can be an interface that enables the communication between a user (medical professional) viewing and providing inputs into an interaction interface and other medical professionals. The interaction interface may also enable the communication between a user and the radiotherapy treatment planning computer model where the user can instruct the radiotherapy treatment planning computer model regarding how to generate/optimize a treatment plan for the patient.

In some embodiments, the analytics server may monitor the communications between the medical professionals and/or the medical professional and the radiotherapy treatment planning computer model. As discussed herein, the medical professional can use a variety of methods to interact with the interaction interface, such as text, audio, video, and the like.

At step **204,** the analytics server may receive, from the radiotherapy treatment planning computer model, an indication associated with the radiotherapy planning attribute.

The monitored communications may include one or more radiotherapy planning attributes associated with a radiotherapy treatment of a patient. These radiotherapy planning attributes may be captured by the analytics server (e.g., as they are being inputted by the medical professional). The analytics server then transmits the radiotherapy planning attribute to a machine learning language processing model and/or the radiotherapy treatment planning computer model. As used herein, the machine learning language processing model may be a large language model that has been trained to ingest communications between multiple parties in light of the RTTP planning and provide contextual data. The machine learning language processing model can be or include a neural network, such as a neural network with a transformer architecture. As discussed herein, the machine learning language processing model may ingest the conversation inputted by one or more medical professional, such that it can provide a response that is relevant to the conversation and/or the inputted material. By presenting data that is relevant to the context of what is inputted by users, the analytics server may provide results/predictions that improve generic chatbot outputs.

Non-limiting examples of a radiotherapy planning attribute may include patient information (e.g., patient demographics, such as age, gender, medical history, previous treatments and outcomes, current health status and any comorbidities, tumor characteristics, tumor type and histology, tumor size, shape, and location, tumor stage, and grade, presence of metastases), imaging data (e.g., CT, MRI, and PET scans, tumor and organ delineations, spatial relationships between tumor and surrounding tissues), dose distribution (e.g., prescribed dose to the tumor (target volume), dose constraints for surrounding critical organs and tissues, dose-volume histograms (DVHs)), field geometry (e.g., beam angles and orientations, field sizes and shapes, MLC (multi-leaf collimator) settings), optimization parameters (e.g., treatment modalities, such as IMRT, VMAT, proton therapy, optimization objectives, and constraints, weighting factors for different treatment goals), technical specifications (e.g., equipment capabilities and limitations, treatment machine calibration and settings, software version and capabilities), and/or clinical protocols (e.g., standard treatment protocols and guidelines, protocol-specific dose constraints and objectives, and the like.

The radiotherapy planning attributes may collectively contribute to developing a comprehensive and effective radiotherapy treatment plan tailored to the individual patient's needs and specific clinical scenarios.

The radiotherapy planning attribute may be inputted via a variety of methods. For instance, the interaction interface may enable a form of communication similar to a conversation between two humans that can involve back-and-forth exchanges of messages. In this case, the participants may be expert users who are medical professionals, such as an oncologist or other clinician, and an AI agent that implements the machine learning language processing model to ingest inputs from the discussion and provide the outputs of the machine learning language processing model (e.g., the machine-readable code).

In some implementations, the interaction interface can simulate an instant messaging application conversation between the medical professional(s) and the machine learning language processing model. For example, a user can input text into the interaction interface. The interaction interface can be a feed (e.g., a user feed). The text can be or include patient attributes for a patient receiving radiotherapy treatment. Moreover, the text can be requested for visualizations that are related to a patient's treatment plan (or proposed treatment plan). In a non-limiting example, the input may be "I would like to see DVH for John Smith, but highlight the OARs in blue."

In some embodiments, the analytics server may receive an input indicating an identifier (e.g., a name) of the patient via the user interface or the platform provided. Responsive to receiving the input, the analytics server can retrieve patient data (e.g., one or more patient attributes) regarding the patient from non-transitory memory containing database records of the patient database. Examples of patient attributes the analytics server may retrieve include computed tomography (CT) scans of the patient or a tumor of the patient, images of the patient or a tumor of the patient, previously collected patient attributes of the patient, such as data collected from previous health tests, among others. The analytics server may also query a treatment planner and receive a treatment plan (or proposed treatment plan) for the identified patient.

After transmitting the radiotherapy treatment planning attribute to the radiotherapy treatment planning computer model, the model may transmit an indication back to the analytics server. As discussed herein, the radiotherapy treatment planning computer model may refer to any computer model that is configured to receive attributes associated with a radiotherapy treatment plan and generate a treatment plan for a patient. The radiotherapy treatment planning computer model may also be referred to herein as a plan optimizer. The analytics server may periodically communicate with the radiotherapy treatment planning computer model in order to validate the inputs provided by the medical professionals.

The indication may include a message that the data received from the user is missing and/or inadequate. In some embodiments, the indication may convey that the radiotherapy planning attribute inputted by the medical professional would yield results that would violate the plan objectives (e.g., thresholds identified by an oncologist).

Additionally, or alternatively, the analytics server may use various defined (e.g., pre-determined) rules and thresholds to evaluate the radiotherapy planning attribute and/or the indication received. For instance, various radiotherapy planning attributes can correspond to various thresholds defined by a set of pre-defined rules and/or medical professionals (e.g., plan objectives identified by an oncologist or a radiologist). The analytics server may validate the inputs against those rules. In some embodiments, while the radiotherapy planning attributes may be within the defined ranges, the resultant treatment plan may include one or more attributes that are outside tolerable thresholds. For instance, while a prescribed attribute may be within reasonable bounds (below a defined threshold), it might result in a dosage to an OAR that is more than the tolerable threshold. The analytics server may also validate the treatment plans generated as a result of using the attributes provided by the medical professional.

If the indication does not satisfy any thresholds, the method **200** may move to the step **208** where the method **200** ends until a new radiotherapy planning attribute has been received from the user. If, however, the indication does indeed satisfy the threshold (indicating that the user has inputted a radiotherapy treatment planning attribute that can be improved or yields unacceptable results), the method **200** may move to the step **210.**

At step **210,** the analytics server may execute a machine-learning language processing model to generate contextual data associated with the one or more radiotherapy planning attributes. The machine-learning language processing model may be specially trained to ingest patient data/attributes along with one or more radiotherapy planning attributes and generate contextual data associated with the one or more radiotherapy planning attributes.

Prior to executing the machine-learning language processing model, the analytics server may generate or train the machine learning language processing model. The analytics server can generate or train the machine learning language processing model using supervised learning, unsupervised learning, or semi-supervised learning techniques.

The analytics server may first generate a training dataset corresponding to previously treated patients and/or test patients. Accordingly, the training data set may include different sentences or paragraphs of text that correspond to the radiotherapy treatment of patients, treatment visualizations, and the corresponding attributes used to generate the treatments. In some embodiments, the training data may be divided into four categories: (1) patient data, (2) treatment data, (3) treatment visualization and its attributes, and (4) inputted radiotherapy treatment planning attributes. As a result, the machine learning language processing model may learn how to correlate a radiotherapy treatment planning attribute to its one or more attributes of the treatment plan.

The underlying code may also include pertinent libraries used by one or more radiotherapy treatment planning computer models (also included within the training dataset). Different libraries may contain standardized planning language and guideline and references to generate the treatment plans. Moreover, guidelines for a specific radiotherapy machine implementing the treatment may be used to train the model, such that the context data is tailored to the specific radiotherapy machine that is going to be used to treat the patient. Additionally, in some embodiments, clinic specific rules and guidelines may be ingested by the model to train itself, such that the resultant predictions are specific to a particular clinic.

In some embodiments, the analytics server may train the machine-learning language processing model using the training dataset. The machine-learning language processing model may ingest the training dataset and learn how to provide contextual data associated with one or more radiotherapy treatment planning attributes.

In some embodiments, the analytics server may use a general-purpose machine-learning language processing model and utilize an in-context learning paradigm. In-context learning may refer to the model's ability to adapt and generate responses based on the immediate context of a given interaction. In this approach, the model may act without relying on external memory or updates to its parameters. In this approach, the model may utilize the provided prompt (including the radiotherapy planning attribute) to infer the medical professional's intent or context of a conversation among multiple medical professionals.

Additionally, or alternatively, the analytics server may train a specially trained version of the machine-learning language processing model by using a transfer learning technique. For instance, the analytics server may utilize a general-purpose machine-learning language processing model and continuously optimize the model's network parameters using a specialized training set containing examples of contextual data used to guide a medical professional.

After the machine-learning language processing model has been properly trained, the analytics server may execute the model to identify contextual data associated with the user's input received in the step **212.** The analytics server may present a graphical component (e.g., a pop-up window or a portion of the interaction interface) that displays the contextual data generated by the machine-learning language processing model.

In some embodiments, the AI agent may present a series of comments and questions providing contextual data associated with the input and/or subsequent inputs by the user. The contextual data may represent any data that provides insights regarding treatment planning. In particular, the contextual data may provide additional data associated with the input received from the user. In some other embodiments, the contextual data may include and then be used to provide information associated with the category of the input provided by the user.

The scope of the contextual data may encompass the entire treatment planning system (TPS), enabling user interaction across all workspaces. Additionally, or alternatively, the scope may be limited to specific workspaces. Therefore, the AI agent may only be enabled by the analytics server in situations where the user is working outside a particular workspace. For instance, whether the AI agent is implemented by the analytics server may depend on the subject matter of the discussion between the medical professionals or the subject matter of what the user is working on. In some embodiments, the AI agent's scope may include critical areas such as prescription and prescription enhancement, actual planning, and plan review (including potential plan refinement). This broad scope ensures that the AI agent can provide comprehensive support throughout the entire treatment planning process.

The machine-learning language processing model may also consider all the information received from the interaction interface. For instance, the AI agent's responses may consider all (or at least a portion) of the entire communication between the medical professionals or other inputted provided by the medical professional. Any information the analytics server receives (e.g., from the user via the interaction interface) might trigger a reaction (e.g., an intervention and outputting of data by the AI agent), leading to actions visible to the user. These actions could involve adding text to a dialog between the user and the AI agent, which could appear in a chat window, a modal dialog, or as synthesized speech, and the like. For example, the AI agent might provide real-time feedback, offer suggestions, or answer questions posed by the user.

In some embodiments, the machine-learning language processing model may generate visual data that can provide contextual data for the user. For instance, the machine-learning language processing model can generate a simulated medical image and highlight an organ or region of interest, change the image view to a specific slice, and/or overlay additional information on any visualization of the treatment plan. In some embodiments, these visual cues can help the user better understand and navigate complex treatment data. In a non-limiting example, the AI agent may present a DVH graph to the user along with text that explains what is being shown to the user.

In some embodiments, the AI agent could also proactively interrupt the user's communication when necessary. For instance, if the user is about to proceed with an action that might lead to suboptimal outcomes, the AI agent could interject with clarifying questions or additional guidance. This feature may ensure that potential errors are caught early, and the user receives the necessary support to make informed decisions.

In some embodiments, the contextual data outputted may correspond to guidelines and instruction manuals, e.g., by integrating a verified database into its generative process. Various electronic documents (e.g., clinic-specific guidelines, instruction manuals, treatment planning instruction manuals, treatises, and the like) may be used during training the models discussed herein. As a result, a relevant electronic document may be outputted for the benefit of the user. For instance, the analytics server and/or the model may first identify the contextual information as discussed herein and then cross-reference the response with a database containing various electronic document (e.g., trusted guidelines and instruction manuals or other treatment planning-related documents). The final output provided to the user may then consists of specific excerpts or sections from these verified electronic documents, ensuring that the information is reliable and grounded in established clinical standards, minimizing the risks of variability that can arise from purely model-generated responses.

In a non-limiting example, the output presented to the user may include an electronic document (e.g., a copy of the treatment planning guidelines that is specific to the clinic) along with a text description of other contextual data. The analytics server may visually highlight a portion of the electronic document, such that the user is directed towards the relevant section for further review and analysis of the clinic-specific protocols identified as contextually important.

By integrating these capabilities, the machine-learning language processing model aims to enhance the overall efficiency and accuracy of the treatment planning process, reducing the burden on human instructors and allowing users to achieve a higher level of expertise more quickly.

In some embodiments, the machine-learning language processing model can be tailored (fine-tuned) to tailor its outputs based on the context of the input received from the user and/or the context of the user's involvement. For instance, the AI agent may have different "modes" where the reactions (outputted content by the machine-learning language processing model) are tailored in accordance with the AI agent's modes. In some embodiments, the level of guidance provided by the machine-learning language processing model can be adjusted by user-settings and/or in accordance with the mode (e.g., guiding mode, training mode, and/or the mentoring mode). In some embodiments, machine-learning language processing model may identify an appropriate mode for the user in accordance with the user's attributes (e.g., profile information).

Training mode: In this mode, the machine-learning language processing model may provide an AI agent that provides detailed instructions at every step for the user. The AI agent may also provide a commentary for every user action (or propose alternative actions to the user if these actions can improve the quality of the plan). In the training mode, the AI agent can also ask questions that help the user prepare/orient for the next task (and evaluate the answers). In some embodiments, the AI agent detects the knowledge level of the user based on the inputs the user provides and thus the machine-learning language processing model may configure the AI agent to communicate to the user at a level that raises the knowledge level of the user. In that way, the machine-learning language processing model may provide incremental improvements and avoid communicating at a level that the user would not be able to comprehend.

Guiding mode: In this mode, the machine-learning language processing model may adopt a more passive role, allowing users to work more independently without providing feedback for every action. There are specific predefined points in the treatment planning workflow where the AI agent will intervene, such as during the planning approval stage. At these points, the AI agent might provide suggestions on what to check or do before final approval. Additionally, the AI agent may prompt the user with questions, such as "Can I do this for you?" and then explain the action and expected outcomes. Upon the user's approval, the analytics server may execute the action (or instruct another processor to execute the action), thereby functioning as a generative tool that leverages the automatic features of the treatment planning system.

Mentoring mode: In this mode, the machine-learning language processing model may only provide an output when the user is asking something or otherwise engages the AI agent (e.g., by inputting a desire for the AI agent to intervene). When the analytics server receives an indication that the user has requested, the analytics server may instruct the machine-learning language processing model to generate contextual data (e.g., suggestions) for the user. For instance, the AI agent may provide alternative solutions or ask questions for the user that allow the user to improve the action.

Trainer mode: In this mode, the analytics server may monitor the user actions to train the machine-learning language processing model. For instance, the analytics server may monitor how a human expert-level trainer generate a treatment plan (e.g., monitor the inputs provided by the user). The inputs monitored may then be transmitted to the machine-learning language processing model, such that the machine-learning language processing model learns or fine-tunes its training to clinic-specific content, such as actions of the user and/or the reasoning behind the actions, thus enhancing the reasoning capability of the machine-learning language processing model. The machine-learning language processing model may analyze the human trainer's input or the input from the trainer's discussion with another user to further train itself.

The implementation of the method **200** may use a system architecture that is structured using multi-level machine-learning language processing model prompting, where the initial prompt determines the appropriate action based on the current input. This could involve taking various actions, such as doing nothing, retrieving additional information, performing image analysis with the MM-LLM model, and/or initiating a dialog with the user. In some embodiments, the first-level prompt produces a structured data element, such as a JSON object, that encodes the type of action and requires additional information. These actions can form a chain, with each action providing more information until communication with the user is deemed necessary. At this stage, a special machine-learning language processing model prompt may be used to generate the text (or other output) to be presented to the user. User-defined settings, such as the requested mode, can influence these decisions. Additionally, in order to determine some actions to be taken, the analytics server may use traditional algorithmic logic (such as if-else statements) to evaluate collected information in predefined ways, for example, to terminate data gathering after a certain number of actions.

In some embodiments, the analytics server may use prompt engineering to train and/or efficiently implement the machine-learning language processing model. For instance, specially designed prompt patterns may be used to guide the AI agent's output. The prompts can be infused with various types of data to influence the generated sequences. For example, patient-related data can be included to affect the decision-making of the user or to provide adequate information to the user. In some embodiments, an agent mode/setting can determine the level of guidance provided. This tailored input can ensure that the system's responses are relevant and contextually appropriate for the user and the treatment being planned by the user.

In some embodiments, the analytics server may tune the foundation model. The analysis server may enhance the performance of the machine-learning language processing model by continuing its training with an additional dataset tailored to the specific tasks. This fine-tuning can involve incorporating general guidelines on treatment planning and critical considerations at different planning stages. By training the machine-learning language processing model with this specific information, the machine-learning language processing model may become better suited for providing accurate and relevant guidance in the educational context.

In some embodiments, the machine-learning language processing model may be integrated with other tools (e.g., software products or computer models). The analytics server may augment the capabilities of the machine-learning language processing model using by integrating the predictions/generations of the machine-learning language processing model with other computer models or downstream software applications. For instance, integrating a specific MM-LLM model that converts images into text may allow the machine-learning language processing model to provide instructions based on patient geometry or planned dose distribution. In some embodiments, users can also highlight regions of interest in medical images to ask more specific questions. Additionally, the analytics server can retrieve data from educational databases or standard TPS-related databases, enhancing its ability to provide comprehensive and informed guidance. These methods collectively enhance the effectiveness of the machine-learning language processing models in the overall treatment planning system, ensuring that users receive precise, context-aware assistance tailored to their specific needs and scenarios.

In some embodiments, the analytics server may monitor all interactions with the machine-learning language processing model and periodically retrain the machine-learning language processing model in order to improve its accuracy. In some embodiments, the analytics server may incorporate a feature designed to collect information for training the next generation of machine-learning language processing models used to implement the AI agent. This feature may record user actions and communications during sessions led by a human planner or involving the AI agent.

Additionally, annotations might be added to highlight specific aspects of the data or correct the behavior of previous-generation AI agents. For instance, expert planners (or otherwise human or machine reviewers) could also provide annotated planning sessions where the sessions between the AI agent and users are annotated and/or labeled. The annotated data may be augmented via training data, such as examples with new feature releases, new guidelines, and the like. Once the training data is aggregated, the training data can be shared between clinics as part of inter-clinic knowledge transfer, where different machine-learning language processing models are trained using the new knowledge and then fine-tuned or customized for clinic-specific reasons.

In some embodiments, the analytics server may also utilize predefined educational training cases, where each patient case has been previously planned by an expert, to retrain the machine-learning language processing model. For instance, after the re-training, the machine-learning language processing model may guide new users to replicate similar planning processes using these cases.

While some of the embodiments discussed herein refer to the machine-learning language processing model as a single model, other embodiments may use multiple specialized models. These models may be each tailored for different purposes, such as various treatment sites, treatment modalities, or levels of interaction (e.g., training mode vs. guiding mode). In some embodiments, the analytics server may include logic to select the appropriate agent based on the situation. Additionally or alternatively, a single agent could access multiple machine-learning language processing models or different sets of tools and prompt templates to adapt to diverse educational needs.

After presenting the contextual data predicted by using the methods and systems discussed herein, the analytics server may provide an additional input element on the user interface. Using the additional input element, the medical professional may provide an input corresponding to the quality of the data. That is, the medical professional may provide input regarding whether the contextual data was helpful. The analytics server may then re-train or re-calibrate the machine-learning language processing model using the input received from the medical professional.

In some embodiments, the user may adjust the radiotherapy treatment plan in accordance with the user's input or a suggested input received from the AI agent.

For example, the user viewing the radiotherapy treatment plan at the user interface can provide an input (e.g., a text input or a selection of a button) at the user interface that would yield unacceptable results. Responsive to receiving an indication that the input would yield unacceptable results, the analytics server can execute the machine learning language processing model using the input to guide the user. For instance, the AI agent might output, "here is the DVH graph if we use your previous input. As you can see, this portion of the DVH does not seem right. Let's lower the OAR dosage to 5 Gy." As a result, the user may approve lowering the OAR dosage to 5 Gy and then the analytics server may instruct the plan optimizer computer model to generate a new RTTP using the new attribute (e.g., 5 Gy threshold) and display the results.

In a non-limiting example, as illustrated in an example **300,** a user **340** may be interacting with a treatment planning system **320** via a user interface **320A,** such as the interaction interface discussed herein. The analytics server may monitor the user's activities and attribute inputted by the user **340.** When the user **340** inputs an attribute that would yield a radiotherapy treatment plan that does not satisfy a threshold, the analytics server may execute the AI agent **330** and present guidance via the user interface **320A.** The guidance may include specific knowledge stored within an education database **310.**

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc., may be passed, forwarded, or transmitted via any suitable means, including memory sharing, message passing, token passing, network transmission, etc.

The actual software code or specialized control hardware used to implement these systems and methods is not limited to the claimed features or this disclosure. Thus, the operation and behavior of the systems and methods were described without reference to the specific software code, and it is understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate the transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disc, and Blu-ray disc, where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the spirit or scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope and spirit being indicated by the following claims.

## Claims

1. A method comprising:
monitoring, by at least one processor, a radiotherapy treatment planning user interface comprising an interaction interface to identify one or more radiotherapy planning attributes associated with a radiotherapy treatment of a patient via a radiotherapy treatment planning computer model;
receiving, by at least one processor from the radiotherapy treatment planning computer model, an indication associated with the one or more radiotherapy planning attributes; and
responsive to the indication satisfying a threshold:
executing, by at least one processor, a machine-learning language processing model to generate contextual data associated with the one or more radiotherapy planning attributes; and
presenting, by at least one processor, the contextual data within the radiotherapy treatment planning user interface.

2. The method of claim 1, wherein the indication corresponds to missing data.

3. The method of claim 1 or 2, wherein the indication corresponds to the one or more radiotherapy planning attributes causing at least one attribute of the radiotherapy treatment violate a plan objective.

4. The method of claim 1, 2 or 3, wherein presenting the contextual data corresponds to outputting an electronic document corresponding to at least one of an instruction manual, a setting or guideline specific to the radiotherapy treatment planning computer model, or a treatment planning guideline.

5. The method of any one of claims 1 to 4, wherein the contextual data is specific to a clinic implementing the radiotherapy treatment of the patient.

6. The method of any one of claims 1 to 5, wherein the contextual data comprises a visualization of the radiotherapy treatment of the patient.

7. The method of any one of claims 1 to 6, further comprising:
recalibrating, by at least one processor, the machine-learning language processing model using an input corresponding to a quality of the contextual data.

8. A non-transitory computer-readable medium having non-transitory instructions that when executed, cause a processor to:
monitor a radiotherapy treatment planning user interface comprising an interaction interface to identify one or more radiotherapy planning attributes associated with a radiotherapy treatment of a patient via a radiotherapy treatment planning computer model;
receiving, by at least one processor from the radiotherapy treatment planning computer model, an indication associated with the one or more radiotherapy planning attributes; and
responsive to the indication satisfying a threshold:
execute a machine-learning language processing model to generate contextual data associated with the one or more radiotherapy planning attributes; and
present the contextual data within the radiotherapy treatment planning user interface.

9. The non-transitory computer-readable medium of claim 8, wherein the instructions further cause the processor to:
recalibrate the machine-learning language processing model using an input corresponding to a quality of the contextual data.

10. A system comprising:
a machine-learning language processing model; and
a processor in communication with the machine-learning language processing model, the processor configured to:
monitor a radiotherapy treatment planning user interface comprising an interaction interface to identify one or more radiotherapy planning attributes associated with a radiotherapy treatment of a patient via a radiotherapy treatment planning computer model;
receiving, by at least one processor from the radiotherapy treatment planning computer model, an indication associated with the one or more radiotherapy planning attributes; and
responsive to the indication satisfying a threshold:
execute the machine-learning language processing model to generate contextual data associated with the one or more radiotherapy planning attributes; and
present the contextual data within the radiotherapy treatment planning user interface.

11. The non-transitory computer-readable medium of claim 8 or 9, or the system of claim 10, wherein the indication corresponds to missing data.

12. The non-transitory computer-readable medium of claim 8, 9 or 11, or the system of claim 10 or 11, wherein the indication corresponds to the one or more radiotherapy planning attributes causing at least one attribute of the radiotherapy treatment violate a plan objective.

13. The non-transitory computer-readable medium 8, 9, 11 or 12, or the system of claim 10, 11 or 12, wherein presenting the contextual data corresponds to outputting an electronic document corresponding to at least one of an instruction manual, a setting or guideline specific to the radiotherapy treatment planning computer model, or a treatment planning guideline.

14. The non-transitory computer-readable medium 8, 9, 11, 12 or 13, or the system of claim 10, 11, 12 or 13, wherein the contextual data is specific to a clinic implementing the radiotherapy treatment of the patient.

15. The non-transitory computer-readable medium 8, 9, 11, 12, 13 or 14, or the system of claim 10, 11, 12, 13 or 14, wherein the contextual data comprises a visualization of the radiotherapy treatment of the patient.
